# EUROPEAN PATENT APPLICATION

(11) **EP 4 257 968 A1**
(43) Date of publication of application: **11.10.2023**
(21) Application number: 23164953.4
(22) Date of filing: 29.03.2023
(51) Int. Cl.: G01N 27/74

(54) **GAS MEASURING DEVICE**

(30) Priority: 05.04.2022 JP 2022062885
(71) Applicant: Sintokogio, Ltd., Nagoya-shi, Aichi 450-6424 (JP); National University Corporation TOYOHASHI UNIVERSITY OF TECHNOLOGY, Toyohashi-shi, Aichi 441-8580 (JP)
(72) Inventor: MIZUTANI, Manase, Nagoya-shi, Aichi, 450-6424 (JP); SUZUKI, Yoshihisa, Nagoya-shi, Aichi, 450-6424 (JP); NODA, Toshihiko, Toyohashi-shi, Aichi, 441-8580 (JP); SAWADA, Kazuaki, Toyohashi-shi, Aichi, 441-8580 (JP)
(74) Representative: Hoffmann Eitle

(57) **Abstract**

A gas measuring device includes: a magnetic field generation unit configured to generate a magnetic field; and a gas sensor configured to detect a gas that has passed through the magnetic field generated by the magnetic field generation unit.

## Description

### CROSS-REFERENCE TO RELATED APPLICATION

This application is based on Japanese Patent Application No. 2022-062885 filed with Japan Patent Office on April 5, 2022, the entire contents of which are hereby incorporated by reference.

### TECHNICAL FIELD

The present disclosure relates to a gas measuring device.

### BACKGROUND

Japanese Unexamined Patent Publication No. 2015-127642 discloses a gas detection device that includes a filter made of fibrous activated carbon and a MEMS gas sensor. The gas detection device has a function of screening gas components that are detection targets and gas components that are not detection targets from each other by causing the filter to adsorb miscellaneous gases that are not detection targets. The adsorption capacity of the filter decreases with the adsorption of the miscellaneous gases. For this reason, the gas detection device has a heater for regenerating the adsorption capacity of the filter. The filter heated by the heater desorbs the adsorbed gas. Accordingly, the adsorption capacity of the filter is regenerated.

### SUMMARY

The gas detection device disclosed in Japanese Unexamined Patent Publication No. 2015-127642 requires periodical heat cleaning of the filter. However, the gas detection device cannot detect a gas while the heat cleaning is being performed. The present disclosure provides a gas measuring device that can continuously use a screening function for gas components.

A gas measuring device according to an aspect of the present disclosure includes: a magnetic field generation unit configured to generate a magnetic field; and a gas sensor configured to detect a gas that has passed through the magnetic field generated by the magnetic field generation unit.

In this gas measuring device, the gas to be measured by the gas sensor passes through the magnetic field generated by the magnetic field generation unit. When the gas passes through the magnetic field, an attractive force due to the magnetic field acts on a paramagnetic gas. A repulsive force due to the magnetic field acts on a diamagnetic gas. For this reason, the gas measuring device can control a traveling direction of the gas according to the magnetic properties of the gas, and thus a screening function for gas components can be exhibited. Since the magnetic field acts indirectly on the gas, the screening function does not deteriorate even after continuous use. Therefore, this gas measuring device can continuously use the screening function for gas components.

In an embodiment, the magnetic field generation unit may be made of a permanent magnet. In a configuration in which the magnetic field generation unit is made of the permanent magnet, the magnetic field is generated without using electricity, and thus the screening function for gas components can be continuously used with a simple configuration.

In an embodiment, the gas measuring device may further include a power supply unit. The magnetic field generation unit may be a coil. The power supply unit may energize the coil. The coil generates the magnetic field when energized. Therefore, in a configuration in which the magnetic field generation unit is a coil energized by the power supply unit, it is possible to switch a state in which gas components are screened and a state in which gas components are not screened by interrupting the energization.

In an embodiment, the gas measuring device may further include a signal generation unit, a control unit, an acquisition unit, and an output unit. The signal generation unit outputs a synchronization signal for determining a timing. The control unit controls the power supply unit on the basis of a control signal for determining a magnitude of a voltage or current and the synchronization signal such that the voltage or current having the magnitude determined with the control signal is applied to the coil at the timing determined with the synchronization signal. The acquisition unit acquires a detection value of the gas sensor at the timing determined with the synchronization signal. The output unit outputs the detection value and the control signal in association with each other. In this case, the gas measuring device can output the detection value and the synchronization signal in association with each other.

In an embodiment, the gas measuring device may further include a determination unit configured to determine a gas type on the basis of a pre-acquired relationship among the gas type, the detection value, and the control signal, and the detection value and the control signal output from the output unit. In this case, the gas measuring device can determine the gas types of a mixed gas including a paramagnetic gas component and a diamagnetic gas component of which traveling directions are different from each other.

In an embodiment, the gas measuring device may further include a plurality of gas sensors including the gas sensor. The plurality of gas sensors includes different gas sensors capable of detecting a gas type different from a gas type detected by the gas sensor. In this case, the gas measuring device can improve gas detection accuracy compared to a gas measuring device having a gas sensor capable of detecting a single type of a gas.

In an embodiment, the gas measuring device may further include a plurality of gas sensors including the gas sensor. The plurality of gas sensors is disposed in a direction of the magnetic field generated by the magnetic field generation unit. An attractive force in the direction of the magnetic field acts on the traveling direction of the paramagnetic gas. A repulsive force in the direction of the magnetic field acts on the traveling direction of the diamagnetic gas. For this reason, the plurality of gas sensors can detect a plurality of types of gas components screened in the direction of the magnetic field.

The present disclosure provides a gas measuring device that can continuously use a screening function for gas components.

### BRIEF DESCRIPTION OF THE DRAWINGS

FIG. 1 is a cross-sectional view showing an example of a gas measuring device according to an embodiment.
FIG. 2 is a schematic view illustrating a traveling direction of a gas passing through a magnetic field generation unit according to the embodiment.
FIG. 3A is a block diagram showing an example of the gas measuring device according to the embodiment. FIG. 3B shows examples of a control signal and a synchronization signal.
FIG. 4A is a schematic view illustrating a change in the traveling direction of the gas due to a strength of a magnetic field. FIG. 4B is another schematic view illustrating a change in the traveling direction of the gas due to a strength of a magnetic field. FIG. 4C is yet another schematic view illustrating a change in the traveling direction of the gas due to a strength of a magnetic field.
FIG. 5A is a cross-sectional view showing a modification example of the gas measuring device according to the embodiment. FIG. 5B is a cross-sectional view showing another modification example of the gas measuring device according to the embodiment.

### DETAILED DESCRIPTION

Embodiments of the present disclosure will be described below with reference to the drawings. In the following description, the same or corresponding elements are denoted by the same reference signs, and redundant description will not be repeated. The dimensional proportions of the drawings do not necessarily match those of the description.

### [Configuration of gas measuring device]

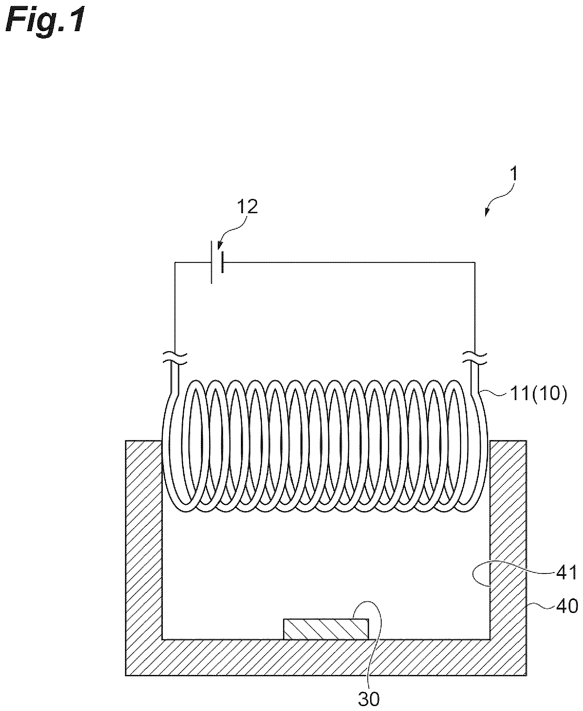

FIG. 1 is a cross-sectional view showing an example of a gas measuring device 1 according to an embodiment. The gas measuring device 1 shown in FIG. 1 is a device for measuring gas components. The gas measuring device 1 may be provided as an electric circuit part. As an example, the gas measuring device 1 is a micro electro mechanical systems (MEMS) device. The gas measuring device 1 includes a magnetic field generation unit 10, a power supply unit 12, a gas sensor 30, and a base member 40.

The base member 40 defines a space therein. The base member 40 is formed of a gas impermeable material. An upper portion of the base member 40 is open, and the base member 40 has an opening that communicates with the space. In the present embodiment, the magnetic field generation unit 10 is a coil 11. The coil 11 is disposed to cover the opening in the upper portion of the base member 40. A gap through which a gas can pass is defined in the coil 11. The coil 11 and the base member 40 are joined together such that there is no gap through which a gas permeates other than the coil 11. Accordingly, the coil 11 and the base member 40 define a gas chamber 41.

The coil 11 is an air-core coil made of a wire wound around an axial direction. As an example, the axial direction of the coil 11 and a direction in which the inside and the outside of the gas chamber 41 communicate with each other are orthogonal to each other. The wire constituting the coil 11 is connected to the power supply unit 12 capable of applying a voltage or current. In this case, a direction of a magnetic field generated in the energized coil 11 is the axial direction.

The gas sensor 30 is provided inside the gas chamber 41. As an example, the gas sensor 30 is provided downstream of the coil 11. The gas sensor 30 is provided on a side on which the gas has passed through the coil 11. As an example, the gas measuring device 1 further includes a plurality of gas sensors 30, 31, and 32 including the gas sensor 30. The gas sensors 31 and 32 are gas sensors of different types from the gas sensor 30, which can detect a gas type different from a gas type detected by the gas sensor 30. Each of the gas sensors 30, 31, and 32 detects a gas that has passed through the coil 11 and adhered to a predetermined surface of each of the gas sensors 30, 31, and 32.

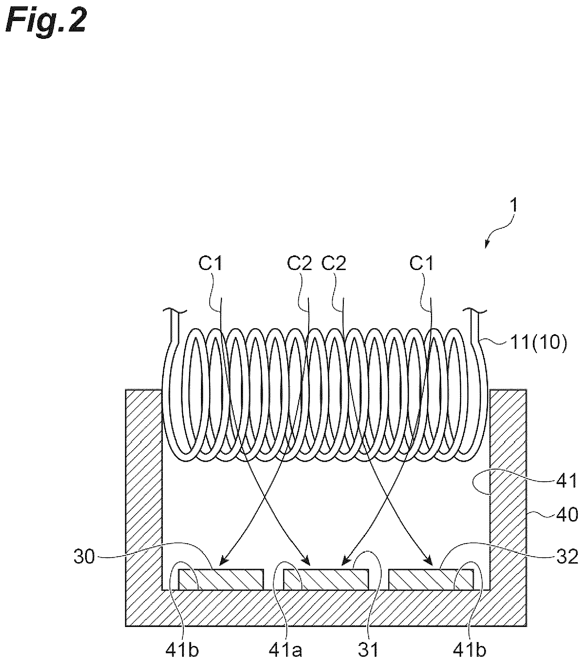

FIG. 2 is a schematic view showing a traveling direction of a gas passing through the coil 11 of FIG. 1. In FIG. 2, the coil 11 generates a magnetic field in the axial direction of the coil 11 as an example. The magnetic flux density in the coil 11 is highest at the center in the axial direction and lowest at both ends in the axial direction. The gas passing through the coil 11 may include a paramagnetic gas component and a diamagnetic gas component. Paramagnetic gases include, for example, oxygen (O₂), nitrogen monoxide (NO), and nitrogen dioxide (NO₂). Diamagnetic gases include, for example, water vapor (H₂O), chlorine (Cl₂), and carbon dioxide (CO₂).

The traveling directions of the paramagnetic gas and the diamagnetic gas are changed by the coil 11. An attractive force due to the magnetic field acts on the paramagnetic gas. A traveling direction C1 of the paramagnetic gas is attracted to a position where the magnetic flux density is high. In the present embodiment, the traveling direction C1 of the paramagnetic gas changes to be attracted to the center of the coil 11 in the axial direction. A repulsive force due to the magnetic field acts on the diamagnetic gas. A traveling direction C2 of the diamagnetic gas is attracted to a position where the magnetic flux density is low. In the present embodiment, the traveling direction C2 of the diamagnetic gas changes to be attracted to both ends of the coil 11 in the axial direction.

As shown in FIG. 2, the gas sensor 31 is provided in a region 41a within the gas chamber 41. The region 41a is set downstream of the center of the coil 11 in the axial direction, for example. The region 41a is a region that the gas attracted to the center of the coil 11 in the axial direction reaches. The gas sensor 30 and the gas sensor 32 are disposed in a pair of adjacent regions 41b with the region 41a interposed therebetween. The pair of regions 41b are set downstream of both ends of the coil 11 in the axial direction. The pair of regions 41b are regions within the gas chamber 41 other than the region 41a. The pair of regions 41b are regions that the gas attracted to both ends of the coil 11 in the axial direction reaches. The region 41a and the pair of regions 41b are arranged in the axial direction of the coil 11. Therefore, the gas sensors 30, 31, and 32 are arranged in the direction of the magnetic field generated by the coil 11.

The gas sensor 31 mainly detects the paramagnetic gas component whose traveling direction C1 is changed to the center of the coil 11 in the axial direction. The gas sensors 30 and 32 mainly detect the diamagnetic gas component whose traveling direction C2 is changed to both ends of the coil 11 in the axial direction.

### [Control circuit of gas measuring device]

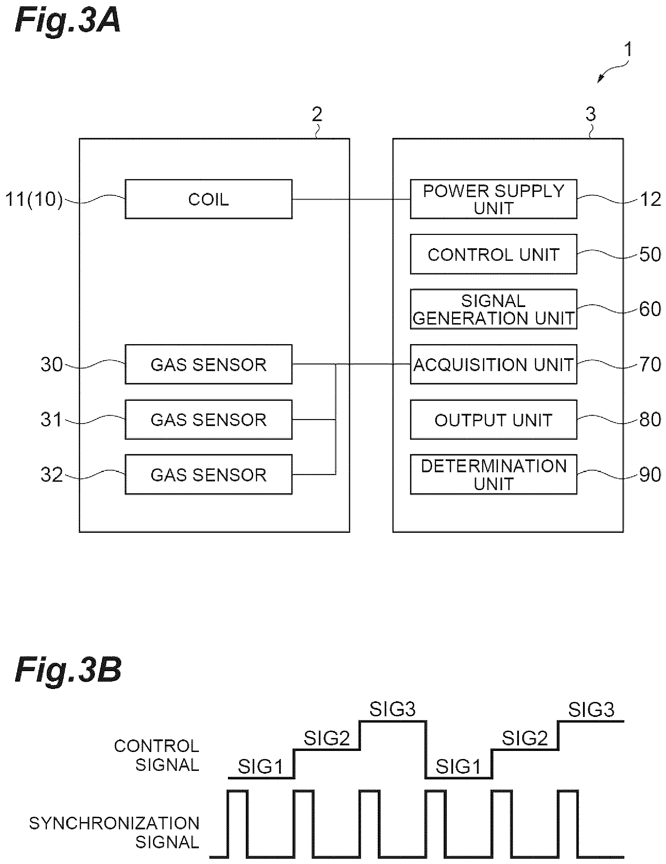

FIG. 3A is a block diagram showing an example of the gas measuring device 1 according to the embodiment. The gas measuring device 1 includes a measuring unit 2 and a circuit unit 3. The measuring unit 2 is an example of a gas measuring device. The circuit unit 3 includes a power supply unit 12, a control unit 50, a signal generation unit 60, an acquisition unit 70, an output unit 80, and a determination unit 90. The circuit unit 3 may be constituted by an electric circuit, for example. The circuit unit 3 may be constituted by a general purpose computer that includes, for example, an arithmetic device such as a central processing unit (CPU), a storage device such as a read only memory (ROM), a random access memory (RAM), a hard disk drive (HDD), a communication device, and the like.

The signal generation unit 60 outputs a control signal and a synchronization signal to the control unit 50 and the acquisition unit 70. The control signal is a signal for determining the magnitude of the voltage or current that is applied to the coil 11 from the power supply unit 12. The synchronization signal is a signal for determining a timing of an operation of each of the control unit 50 and the acquisition unit 70. The control unit 50 controls the power supply unit 12 on the basis of the control signal and the synchronization signal. Specifically, the control unit 50 controls the power supply unit 12 such that the voltage or current having the magnitude determined with the control signal is applied to the coil 11 at the timing determined with the synchronization signal. FIG. 3B shows examples of the control signal and the synchronization signal. In FIG. 3B, the control signal includes three types of signals SIG1, SIG2, and SIG3. SIG1, SIG2, and SIG3 are control signals corresponding to three types of voltage magnitudes. The synchronization signal is, for example, a rectangular wave based on an oscillator such as a crystal oscillator.

The control unit 50 outputs a signal for controlling the power supply unit 12 according to the control signal upon receiving the rectangular wave of the synchronization signal a predetermined number of times. As an example, the control signal that changes in three steps in FIG. 3B is for controlling the magnitude of the voltage in three steps. The magnitude of the voltage may be controlled steplessly. In this case, the control signal exhibits a triangular wave or a sine wave.

The acquisition unit 70 acquires detection values detected by the gas sensors 30, 31, and 32 at the timing determined with the synchronization signal upon receiving the rectangular wave of the synchronization signal a predetermined number of times. Therefore, the acquisition unit 70 can acquire the detection value corresponding to a change in the magnitude of the voltage or current due to the control signal. The predetermined number of times of reception of the rectangular wave when the acquisition unit 70 acquires the detection value may be equal to or greater than the number of times of reception of the rectangular wave when the control unit 50 outputs the control signal. By setting the time at which the acquisition unit 70 acquires the detection value to be later than the time at which the control unit 50 outputs the control signal, it is possible for the acquisition unit 70 to acquire the detection value when the amount of the gas that has passed through the coil 11 becomes large after the magnitude of the voltage or current changes.

The output unit 80 outputs the control signal and the detection value acquired by the acquisition unit 70 in association with each other. The determination unit 90 determines the gas type on the basis of a pre-acquired relationship among the gas type, the detection value, and the control signal, and the detection value and the control signal output from the output unit 80. Combinations of the gas type, the detection value, and the control signal are acquired in advance. Combinations of the gas type, the detection value, and the control signal are stored, for example, as a gas characteristic table. The determination unit 90 refers to the gas characteristic table on the basis of the combination output from the output unit 80 and determines the gas type.

### [Operation of gas measuring device]

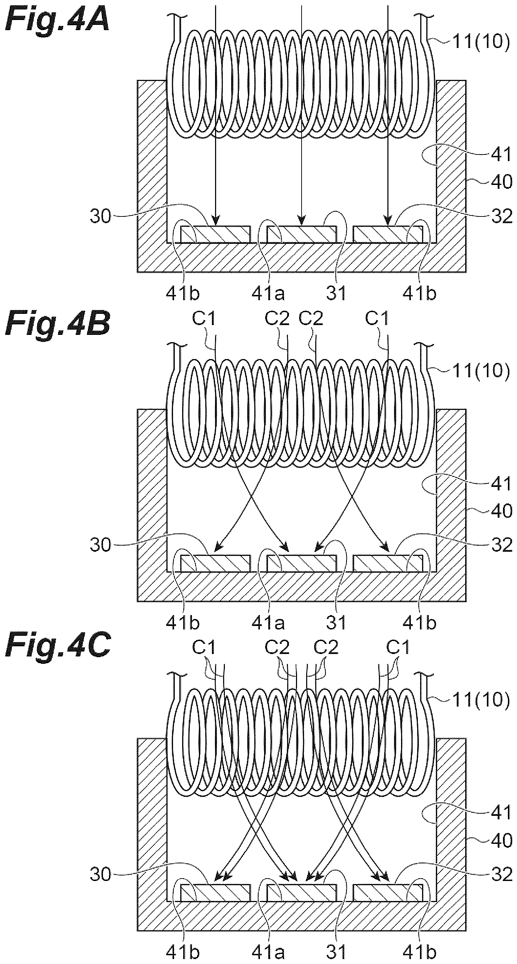

FIGS. 4A to 4C are schematic views illustrating a change in the traveling direction of the gas due to a strength of the magnetic field generated by the coil 11. As an example, a gas that is a detection target is a mixed gas including the paramagnetic gas and diamagnetic gas components. The gas sensors 30, 31 and 32 are disposed downstream of the coil 11. The gas sensor 31 is provided in the region 41a downstream of the center of the coil 11 in the axial direction. The gas sensors 30 and 32 are disposed in the regions 41b set downstream of both ends of the coil 11 in the axial direction.

As shown in FIG. 3B, first, the control unit 50 changes the magnitude of the voltage or current applied to the coil 11 on the basis of the control signal and the synchronization signal for determining the timing. FIG. 4A shows the traveling direction of the gas passing through the coil 11 in a case where the control signal is SIG1. As an example, in a case where the control signal is SIG1, the control unit 50 does not apply a voltage or current to the coil 11. Therefore, a magnetic field is not generated in the coil 11. In a case where the control signal is SIG1, the traveling directions of the paramagnetic gas component and the diamagnetic gas component do not change.

FIG. 4B shows the traveling direction of the gas passing through the coil 11 in a case where the control signal is SIG2. In a case where the control signal is SIG2, the control unit 50 applies a voltage or current to the coil 11. A magnetic field that changes the traveling direction of the paramagnetic gas component into a direction closer to the center of the coil 11 in the axial direction is generated in the coil 11. Therefore, in a case where the control signal is SIG2, the traveling direction of the paramagnetic gas component changes into a direction closer to the center of the coil 11 in the axial direction. The gas sensor 31 mainly detects the paramagnetic gas component. As an example, the gas sensor 31 detects oxygen (O₂) and nitrogen monoxide (NO). The traveling direction of the diamagnetic gas component changes into a direction closer to the both ends of the coil 11 in the axial direction. The gas sensors 30 and 32 mainly detect the diamagnetic gas component compared to the gas sensor 31. As an example, the gas sensors 30 and 32 detect water vapor (H₂O) and chlorine (Cl₂).

FIG. 4C shows the traveling direction of the gas passing through the coil 11 in a case where the control signal is SIG3. In a case where the control signal is SIG3, the control unit 50 applies a larger voltage or current to the coil 11 than in a case where the control signal is SIG2. A stronger magnetic field is generated in the coil 11 than in a case where the control signal is SIG2. Therefore, in a case where the control signal is SIG3, the traveling direction of the paramagnetic gas component changes more greatly into a direction closer to the center of the coil 11 in the axial direction than in a case where the control signal is SIG2. The gas sensor 31 detects more paramagnetic gas component than in a case where the control signal is SIG2. In a case where the control signal is SIG3, the traveling direction of the diamagnetic gas component changes more greatly into a direction closer to both ends of the coil 11 in the axial direction than in a case where the control signal is SIG2. The gas sensors 30 and 32 detect more diamagnetic gas component compared to the gas sensor 31. than in a case where the control signal is SIG2. The gas sensors 30 and 32 detect more diamagnetic gas component than in a case where the control signal is SIG2.

Next, the acquisition unit 70 acquires a first measured value output from the gas sensor 31 and second measured values output from the gas sensors 30 and 32 on the basis of the synchronization signal. The output unit 80 outputs the control signal and the detection value acquired by the acquisition unit 70 in association with each other. The determination unit 90 determines the gas type on the basis of a pre-acquired relationship among the gas type, the detection value, and the control signal, and the detection value and the control signal output from the output unit 80.

The determination unit 90 determines the gas type on the basis of the gas characteristic table, the first measured value and the second measured value acquired by the acquisition unit 70, and the control signal. On the basis of the pre-acquired relationship between the magnitude of the voltage or current indicated by the control signal and the measured value, it is determined that gases that are detection targets include oxygen (O₂), nitrogen monoxide (NO), water vapor (H₂O), and chlorine (Cl₂).

### [Outline of embodiment]

According to the gas measuring device 1, the gases to be measured by the gas sensors 30, 31, and 32 pass through the magnetic field generated by the magnetic field generation unit 10. At this time, an attractive force due to the magnetic field acts on the traveling direction of the paramagnetic gas. A repulsive force due to the magnetic field acts on the traveling direction of the diamagnetic gas. For this reason, the gas measuring device 1 can control the traveling direction of the gas according to the magnetic properties of the gas, and thus the screening function for gas components can be exhibited. Furthermore, since the magnetic field acts indirectly on the gas, the screening function does not deteriorate even after continuous use. Therefore, this gas measuring device 1 can continuously use the screening function for gas components.

In the gas measuring device 1, the energized coil 11 generates a magnetic field. Therefore, in the gas measuring device 1, it is possible to switch a state in which gas components are screened and a state in which gas components are not screened by interrupting the energization.

In the gas measuring device 1, on the basis of the control signal for determining the magnitude of the voltage or current and the synchronization signal, the control unit 50 controls the power supply unit 12 such that the voltage or current having the magnitude determined with the control signal is applied to the coil 11 at the timing determined with the synchronization signal. Then, the acquisition unit 70 acquires the detection values of the gas sensors 30, 31, and 32 at the timing determined with the synchronization signal. In this way, the gas measuring device 1 can output the detection value and the synchronization signal in association with each other.

In the gas measuring device 1, the determination unit 90 determines the gas type on the basis of the pre-acquired relationship, and the detection value and the control signal output from the output unit 80. As a result, the gas measuring device 1 can determine the gas types of the mixed gas including the paramagnetic gas and the diamagnetic gas.

In the gas measuring device 1, the gas sensors 31 and 32 can detect the gas type different from the gas type detected by the gas sensor 30. The gas measuring device 1 can improve gas detection accuracy compared to a gas measuring device having a gas sensor capable of detecting a single type of a gas.

In the gas measuring device 1, a plurality of gas sensors 30, 31, and 32 are disposed in the direction of the magnetic field of magnetic field generation unit 10. An attractive force in the direction of the magnetic field acts on the traveling direction C1 of the paramagnetic gas. A repulsive force in the direction of the magnetic field acts on the traveling direction C2 of the diamagnetic gas. For this reason, in the gas measuring device 1, a plurality of types of gas components screened in the direction of the magnetic field can be detected by a plurality of gas sensors 30, 31, and 32.

### [Modification example]

In the above, various exemplary embodiments have been described, but the present disclosure is not limited to the above exemplary embodiments, and various omissions, substitutions, and modifications may be made.

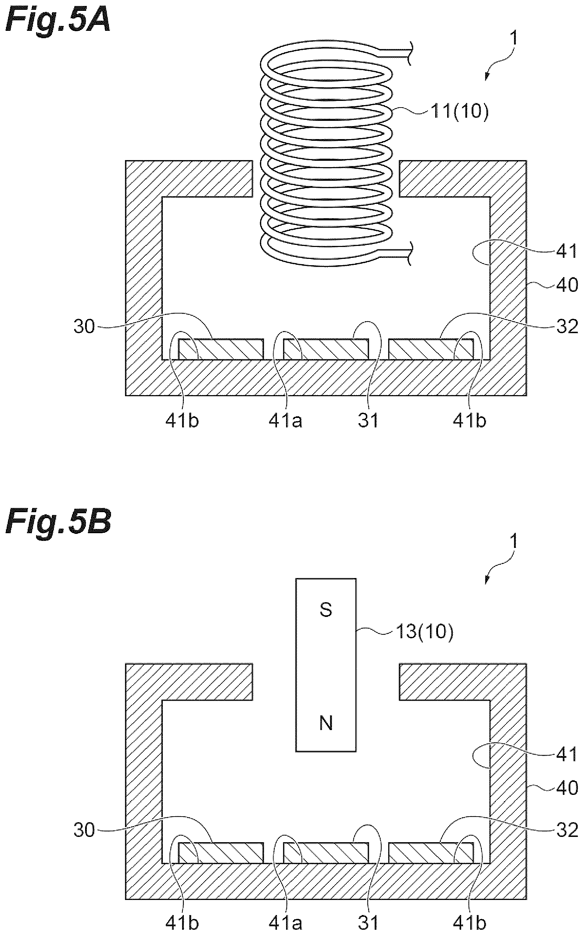

As shown in FIG. 5A, the axial direction of the coil 11 may extend in a direction in which the inside and the outside of the gas chamber 41 communicate with each other. In this case, the magnetic field generated by the coil 11 passes through the gas sensor 31 and the region 41a. The gas sensor 31 mainly detects the paramagnetic gas that is attracted to the coil 11.

As shown in FIG. 5B, the magnetic field generation unit 10 may be a permanent magnet 13. In the permanent magnet 13, an N pole and an S pole may be aligned in a direction in which the inside and the outside of the gas chamber 41 communicate with each other. In this case, the magnetic field generated by the permanent magnet 13 passes through the gas sensor 31 and the region 41a. The gas sensor 31 mainly detects the paramagnetic gas that is attracted to the permanent magnet 13. In a configuration in which the magnetic field generation unit 10 is made of the permanent magnet 13, the magnetic field is generated without using electricity, and thus the screening function for gas components can be continuously used with a simple configuration.

The magnetic field generation unit 10 and the gas sensor 30 may be manufactured by being combined after they are separately formed. The magnetic field generation unit 10 and the gas sensor 30 may be manufactured as a single body.

The signal generation unit 60 may be integrated with the control unit 50. The acquisition unit 70 may be integrated with the output unit 80. The output unit 80 may be integrated with the determination unit 90.

The gas measuring device 1 may be configured not to include the base member 40 and the gas chamber 41. In this case, the gas measuring device 1 is configured such that the magnetic field generation unit 10 is in close contact with the gas sensor 30. The gas measuring device 1 may include M types of gas sensors (M is an integer equal to or greater than 2). In a case where M is 3 or more, the M types of gas sensors may include the same type of sensors. Further, a strength of an electrostatic force of the coil 11 may be controlled in N steps (N is an integer equal to or greater than 2). In this case, the gas measuring device 1 can measure a maximum of M × N combinations of gas types.

The gas measuring device 1 may be configured not to include the determination unit 90. In this case, in the gas measuring device 1, the output unit 80 outputs the measured value and the control signal to the outside. The gas measuring device 1 may acquire in advance the relationship between the measured value and the control signal through simulation. In this case, a strength of the magnetic field generated by the magnetic field generation unit 10 and a force generated between the paramagnetic gas and the diamagnetic gas can be obtained through calculation. The relationship between the measured value and the control signal may be calibrated with the known gas. In this case, the relationship between the control signal and a strength of electrolysis of the magnetic field generation unit 10, and the output characteristics of the gas sensor 30 are calibrated on the basis of the known gas.

## Claims

1. A gas measuring device comprising:
a magnetic field generation unit configured to generate a magnetic field; and
a gas sensor configured to detect a gas that has passed through the magnetic field generated by the magnetic field generation unit.

2. The gas measuring device according to claim 1, wherein the magnetic field generation unit is made of a permanent magnet.

3. The gas measuring device according to claim 1, further comprising a power supply unit,
wherein the magnetic field generation unit is a coil, and
wherein the power supply unit energizes the coil.

4. The gas measuring device according to claim 3, further comprising:
a signal generation unit configured to output a synchronization signal for determining a timing;
a control unit configured to control the power supply unit on the basis of a control signal for determining a magnitude of a voltage or current and the synchronization signal such that the voltage or current having the magnitude determined with the control signal is applied to the coil at the timing determined with the synchronization signal;
an acquisition unit configured to acquire a detection value of the gas sensor at the timing determined with the synchronization signal; and
an output unit configured to output the detection value and the control signal in association with each other.

5. The gas measuring device according to claim 4, further comprising a determination unit configured to determine a gas type on the basis of a pre-acquired relationship, the detection value, and the control signal,
wherein the pre-acquired relationship is a relationship among the gas type, the detection value, and the control signal.

6. The gas measuring device according to any one of claims 1 to 5, further comprising a plurality of gas sensors including the gas sensor,
wherein the plurality of gas sensors includes different gas sensors capable of detecting a gas type different from the gas type detected by the gas sensor.

7. The gas measuring device according to claim 6, wherein the plurality of gas sensors is disposed in a direction of the magnetic field generated by the magnetic field generation unit.
